(19) Europäisches Patentamt
European Patent Office
Office européen des brevets

(11) EP 1 181 350 B1

(12) **EUROPÄISCHE PATENTSCHRIFT**

(45) Veröffentlichungstag und Bekanntmachung des Hinweises auf die Patenterteilung:
**04.05.2005 Patentblatt 2005/18**

(21) Anmeldenummer: **00931218.2**

(22) Anmeldetag: **16.05.2000**

(51) Int Cl.[7]: **C12N 1/26**, C12N 1/20

(86) Internationale Anmeldenummer:
**PCT/EP2000/004365**

(87) Internationale Veröffentlichungsnummer:
**WO 2000/071676 (30.11.2000 Gazette 2000/48)**

(54) **VERWENDUNG VON PIT-EMULSIONEN IN FERMENTATIONSVERFAHREN**

UTILIZATION OF PIT EMULSIONS IN FERMENTATION PROCESSES

UTILISATION D'EMULSIONS PIT DANS DES PROCEDES DE FERMENTATION

(84) Benannte Vertragsstaaten:
**AT BE CH CY DE DK ES FI FR GB GR IE IT LI LU MC NL PT SE**
Benannte Erstreckungsstaaten:
**RO SI**

(30) Priorität: **25.05.1999 DE 19923785**

(43) Veröffentlichungstag der Anmeldung:
**27.02.2002 Patentblatt 2002/09**

(73) Patentinhaber: **Cognis Deutschland GmbH & Co. KG**
**40589 Düsseldorf (DE)**

(72) Erfinder:
- **WEGENER, Matthias**
  **D-40597 Düsseldorf (DE)**
- **MOLITOR, Jean-Pierre**
  **D-40589 Düsseldorf (DE)**
- **DE HAUT, Christian**
  **F-77310 Boissise le Roi (FR)**
- **ABRIBAT, Benoit**
  **F-91490 Dannemois (FR)**
- **ROGGE, Bent**
  **D-40227 Düsseldorf (DE)**

(56) Entgegenhaltungen:
**EP-A- 0 558 112**
**DE-A- 4 140 562**
**US-A- 4 871 665**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 181 350 B1

Printed by Jouve, 75001 PARIS (FR)

## Beschreibung

Die vorliegende Erfindung betrifft die Verwendung von Emulsionen, die nach dem PIT-Verfahren hergestellt werden, in Fermentationsverfahren.

Bei der Synthese komplexer Naturstoffe oder sonstiger organischer Verbindungen, beispielsweise Antibiotika, werden zunehmend mikrobiologische Verfahren eingesetzt. Dabei handelt es sich um eine Stoffumwandlung unter anaeroben oder aeroben Bedingungen, bei der Mikroorganismen, insbesondere aber Bakterien oder Pilze, beteiligt sind. Für derartige Verfahren werden in der Fachwelt verschiedene, nicht immer klar voneinander abgegrenzte Ausdrücke, wie *"Bioconversion"*, "*Biotransformation*" oder "*Fermentation*" verwendet. Der letztere Ausdruck wird auch im Rahmen der vorliegenden Anmeldung für solche Verfahren verwendet, bei denen Mikroorganismen, vorzugsweise Bakterien, zur Umwandlung bzw. Synthese von chemischen Verbindungen verwendet werden.

Für die Entwicklung und Optimierung von Fermentationsprozessen ist insbesondere das Reaktionsmedium, in dem die mikrobiologische Umwandlung stattfindet, von Bedeutung. Das Reaktionsmedium, in aller Regel eine wäßrige Lösung oder Dispersion, beeinflußt insbesondere die Ausbeute und Effizienz des Verfahrens. Die Mikroorganismen benötigen als Nährstoffe Kohlenstoff, Stickstoff und bestimmte Spurenelemente in gebundener Form, z.B. Calcium, Eisen, Phosphor oder Zink, um eine erfolgreiche Metabolisierung zu den gewünschten Produkten möglich zu machen. Weiterhin muß regelmäßig ein bestimmter, meistens enger Temperatur- und pH-Bereich eingehalten werden. Zu weiteren Einzelheiten sei hier auf das Lehrbuch von W. *Crueger*/A. *Crueger,* Biotechnologie - Lehrbuch der angewandten Mikrobiologie, 2. Auflage 1984, R. Oldenbourg Verlag, verwiesen. Insbesondere Kapitel 5 dieses Werkes beschäftigt sich mit den Grundlagen der Fermentationstechnik. Diese Literaturstelle gehört daher auch ausdrücklich zur Offenbarung der vorliegenden Anmeldung. Als Nährstoffe für die Mikroorganismen werden neben energiereichen Zuckern und deren Derivaten in vielen Verfahren zusätzlich natürliche Fette und Öle, sowie Derivate dieser Stoffklassen, wie Glycerin, Glyceride, Fettsäuren oder Fettsäureester eingesetzt. Selbstverständlich dürfen die Kulturmedien keine Inhaltsstoffe aufweisen, die die Metabolisierung der Mikroorganismen negativ beeinflussen können.

Aus der DE 37 38 812 A1 ist beispielsweise ein mikrobielles Verfahren zur Herstellung von alpha-omega-Dicarbonsäuren bekannt, wobei Bakterien des Stamms *Candida tropicalis* Methyllaurat in die gewünschten Dicarbonsäuren umwandeln. Diese Umwandlung findet in einem wäßrigen Medium bei einem pH-Wert von 6,0 und einer Temperatur von 30 °C statt. Das Medium enthält, neben den Mikroorganismen, als Energielieferant Glucose, weiterhin als Emulgator ethoxyliertes Sorbitanmonooleat, Hefeextrakt, Maisquellwasser sowie anorganische N- und P-Quellen. Dem Medium wird dann das Methyllaurat zudosiert. Der Schrift ist kein Hinweis auf den Emulsionstyp zu entnehmen, der sich im Fermenter ausbildet bzw. in dem das Methyllaurat der Fermentationsbrühe zudosiert wird. Aus der EP 0 535 939 A1 ist ein Verfahren zur Herstellung von omega-9-mehrfach ungesättigten Fettsäuren bekannt, wobei in einem wäßrigen Kulturmedium geeignete Mikroorganismen in Gegenwart von Zuckern als Energielieferanten und anorganischen oder organischen Stickstoffquellen, sowie in Gegenwart von Fettsäuremethylestern die gewünschten mehrfach ungesättigten Fettsäuren produzieren. In der US 4,140,562 wird ein Verfahren zur Herstellung von Biopolymeren durch Mikroorganismen in O/W-Emulsionen beschrieben, wobei als Öle pflanzliche Triglyceride eingesetzt werden können. Die Emulsionen werden durch einfache Mischung der Inhaltsstoffe hergestellt.

Es sind aber auch Verfahren bekannt, wo nur Fettstoffe der oben bezeichneten Art als Energielieferanten verwendet werden. Dies ist besonders von wirtschaftlichem Interesse, da derartige Fettstoffe in der Regel preiswerter sind als Zucker, Stärke und ähnliche Verbindungen. Park et al. beschreiben (Park et al., Journal of Fermentation and Bioengineering, Vol. 82, No. 2, 183-186, 1996) einen Fermentationsprozess zur Herstellung von Tylosin, bei dem Mikroorganismen des Stammes *Streptomyces fradiae* in einem wäßrigen Medium verwendet werden, wobei als einzige Kohlenstoffquelle Rapsöl in Ausgangsmengen von etwa 60 g/l enthalten ist.

Bei Fermentationsverfahren spielt außerdem der Sauerstoffgehalt im Medium bzw. der Fermentationsbrühe, eine entscheidende Rolle. Dabei kommt dem Sauerstoff bei aeroben Prozessen die Rolle eines Substrates zu. Entscheidend ist, ob ein für das jeweilige Verfahren ausreichender Sauerstoffübergang von der Gas- in die Flüssigphase, die die Mikroorganismen enthält, stattfinden kann. Ein wichtiger Parameter stellt die spezifische Ausstauschfläche dar, die in der Regel indirekt über den Sauerstoffübergangskoeffizienten $k_{La}$ bestimmt wird (vergl. Literaturstelle *Crueger,* Kapitel 5, Seite 71 ff). Die Einstellung des optimalen Sauerstoffeintrags erfolgt typischerweise durch Rühren der Fermentationsbrühe, wobei der Sauerstoff bzw. die Luft mit der Flüssigkeit vermischt wird und so an den Grenzflächen der Gasaustausch stattfindet. Allerdings kann der erhebliche mechanische Energieeintrag durch starkes Rühren, wie Park et al. ausführen, auch Teile der Kultur zerstören, und so die Ausbeute des Verfahrens verringern. Die abgestorbenen Mikroorganismen werden außerdem selbst weiter abgebaut und können durch die gebildeten Abbauprodukte zu einer Vergiftung der Kultur rühren, die eine wirtschaftliche Produktion unmöglich macht. Aus der Arbeit von Goma und Rols (G. Goma, J.L. Rols, Biotech. Let., Vol 13, No. 1, Seiten 7 bis 12, 1991) ist bekannt daß die Verwendung von Sojaöl in Fermentationsverfahren zur Herstellung von Antibiotika zu einer Verbesserung des Sauerstoffübergangskoeffizienten $k_{La}$ führt, was bei gleichem Energieeintrag (Rühren) zu einem Anstieg der Ausbeute des Gesamtverfahrens führen kann.

Der vorliegenden Erfindung lag nun die Aufgabe zugrunde, Fermentationsverfahren so zu verbessern, daß einerseits preiswerte Kohlenstoffquellen eingesetzt werden können und andererseits eine ausreichende Versorgung der Mikroorganismen mit Sauerstoff gewährleistet ist, ohne daß eine unzulässig hohe mechanische Belastung der Mikroorganismen durch Rühren auftritt. Es sollte eine Weg gefunden werden, den mechanischen Energieeintrag bei Fermentationsverfahren zu minimieren, ohne daß es zu einer Verringerung der Ausbeute kommt. Vorzugsweise soll eine Erhöhung der Ausbeute trotz verringertem Energieeintrag möglich sein.

Es wurde gefunden, daß die Verwendung von speziellen, feinteiligen Öl-in-Wasser (O/W) Emulsionen die obige Aufgabe löst.

In einer ersten Ausrührungsform wird die Verwendung von O/W-Emulsionen in Fermentationsverfahren beansprucht, wobei die Emulsionen mindestens Wasser, Emulgatoren sowie eine Ölphase enthalten und die Ölphase eine oder mehrere Verbindungen, ausgewählt aus den Gruppen

a) der Fettsäurealkylester und/oder
b) der Triglyceride pflanzlichen Ursprungs enthalten,

die Emulsionen nach dem PIT-Verfahren hergestellt werden und eine Tröpfchengröße im Bereich von 50 bis 400 nm aufweisen.

Es ist bekannt, daß Öl-in-Wasser-Emulsionen (O/W), die mit nichtionogenen Emulgatoren hergestellt und stabilisiert sind, bei Erwärmen eine in der Regel reversible Phaseninversion erfahren können, d.h., daß innerhalb eines bestimmten Temperaturintervalls ein Wechsel des Emulsionstyps von O/W zu W/O (Wasser-in-Öl-Emulsion) erfolgt. Da dabei das Öl zur äußeren, kontinuierlichen Phase wird, sinkt die Leitfähigkeit der Emulsion auf Null. Der Mittelwert der Temperaturen zwischen maximaler und gerade auf Null gefallener Leitfähigkeit der Emulsion bei Temperaturerhöhung wird Phaseninversionstemperatur (PIT) und die auf diese Weise hergestellten Emulsionen werden PIT-Emulsionen genannt.

Es ist auch bekannt, daß die Lage der PIT von vielen Faktoren abhängig ist, zum Beispiel von der Art und des Phasenvolumens der Ölkomponente, von der Hydrophilie und der Struktur der Emulgatoren und der Zusammensetzung des Emulgatorsystems.

Wesentlich für die Feinteiligkeit der PIT-Emulsion ist deren Herstellungsverfahren. In der Regel werden die Wasser- und Ölphase mit den Emulgatoren vermischt und danach auf eine Temperatur oberhalb der PIT erwärmt. Die Leitfähigkeit muß dabei auf Null fallen. Anschließend wird die Emulsion wieder auf die Ausgangstemperatur abgekühlt (in der Regel Raumtemperatur, ca. 20°C) . Dabei erfolgt erst durch Überschreiten und das anschließende Unterschreiten der PIT die Ausbildung der erfindungsgemäß verwendeten Emulsion.

Es ist bekannt, daß nur solche PIT-Emulsionen besonders feinteilig sind, welche bei der Phaseninversion eine Mikroemulsionsphase mit niedriger Grenzflächenspannung zwischen Öl und Wasser oder eine lamellare flüssigkristalline Phase ausbilden. Der entscheidende Schritt ist dabei immer die Rückinvertierung bei Abkühlung.

Die DE 38 19 193 A1 offenbart ein Verfahren zur Herstellung niedrigviskoser O/W-Emulsionen mittels der Phaseninversionstechnik. Hierin wird diese Technik auf Mischungen angewandt, die eine Ölkomponente, einen nichtionischen Emulgator und einen Coemulgator in wäßrigem Milieu enthalten. Die Ölkomponente soll dabei aus 50 - 100 Gew.-% spezieller Mono- bzw. Diester, 0 - 50 Gew.-% $C_8$ - $C_{22}$-Fettsäuretriglyceriden und gegebenenfalls 0 - 25 Gew.-% eines Kohlenwasserstofföls bestehen. Die DE 38 19 193 A1 offenbart über die genannten Bestandteile hinaus keine weiteren Komponenten und nennt keinen Verwendungszweck der hergestellten Emulsionen.

Aus der DE 41 40 562 A1 ist ein Verfahren zur Herstellung von O/W-Emulsionen nach dem PIT-Prinzip bekannt, wobei polare Ölkomponenten mit einem Emulgatorsystem, enthaltend nicht-ionische Emulgatoren des HLB-Werte-Bereichs 10 bis 18 in Gegenwart von Coemulgatoren aus der Gruppe der $C_{12-22}$ Fettalkohole und/oder Guerbet-Alkohole über die PIT-Temperatur der Emulsion erwärmt und anschließend wieder abgekühlt werden, wobei feinteilige Emulsionen erhalten werden.

Aus der DE 196 35 553 A1 sind Emulgatorsysteme zur Herstellung von feinteiligen PIT-Emulsionen bekannt, die als wesentliche Bestandteile Fettsäureethoxylate und Partialglyceride enthalten.

Die erfindungsgemäßen Emulsionen zeichnen sich insbesondere durch ihre Feinteiligkeit aus. Die Tröpfchengröße beträgt 50 bis 400 nm. Vorzugsweise liegt die Tröpfchengröße im Bereich von 100 bis 300 nm, insbesondere im Bereich von 180 bis 300 nm und besonders bevorzugt im Bereich von 160 bis 250 nm. Bei den Tröpfchengrößen wird eine Verteilung nach *Gauss* angenommen. Die Messung erfolgt beispielsweise durch Lichtstreuung oder Absorption.

Die Feinteiligkeit der Öltröpfchen führt zu einer großen Oberfläche zwischen Öl- und Wasserphase und ermöglicht so einen schnellen Kontakt zwischen den in der wäßrigen Phase enthaltenen Mikroorganismen und der die Nährstoffe enthaltende Ölphase. Durch die große Oberfläche wird auch der Gasaustausch, insbesondere von Sauerstoff und $CO_2$, vereinfacht. Zusätzlich kann sich die Viskosität der Emulsion und somit des gesamten Fermeritationsmediums verringern.

In der Folge ist es daher möglich die Rührgeschwindigkeit des Fermentationsmediums deutlich zu verringern wodurch eine Erhöhung der Ausbeute des Fermentationsprozeß ermöglicht wird.

Die PIT-Emulsionen werden erfindungsgemäß dem wäßrigen Fermentationsmedium, welches die Mikroorganismen sowie ggf. weitere Hilfs- und Zusatzstoffe enthält, zudosiert. Die Einzelheiten dieses Verfahrens, insbesondere die Geschwindigkeit und Menge der zudosierten Emulsion, ergeben sich aus der Art der Mikroorganismen und des gewählten Fermentationsverfahrens und können vom Fachmann an die spezifischen Gegebenheiten angepaßt werden.

Die PIT-Emulsionen enthalten, neben Wasser noch eine Ölphase, die Verbindungen aus der Gruppe der Fettsäurealkylester a) oder der nativen pflanzlichen Öle und deren Derivate b) enthält. Es handelt sich bei den Gruppen a) und b) um hydrophobe, in Wasser nicht oder nur sehr gering lösliche Verbindungen, die sowohl als Nährstoffe, also Energielieferanten, für die im Fermentationsprozeß eingesetzten Bakterien dienen können, die aber auch Ausgangsstoffe, also Substrate für die durch Biokonversion gewünschten Produkte darstellen können.

Geeignete Ester der Gruppe a) leiten sich insbesondere ab von gesättigten, ungesättigten, linearen oder verzweigten Fettsäuren mit insgesamt 7 bis 23 Kohlenstoffatomen. Es handelt sich also um Verbindungen der Formel (I)

$$R^1\text{-COO-}R^2 \qquad (I)$$

wobei $R^1$ für einen Alkylrest mit 6 bis 22 C-Atomen steht und $R^2$ ein Alkylrest mit 1 bis 4 C-Atomen ist, wobei Methyl- und Ethylreste besonders bevorzugt sind. Am vorteilhaftesten ist der Einsatz von Methylestem. Die Methylester der Formel (I) können auf übliche Weise, z.B. durch Umesterung von Triglyceriden mit Methanol und anschließender Destillation, erhalten werden. Geeignete Fettsäuren sind die Capron-, Heptan-, Capryl-, Perlagon-, Caprin-, Undecan-, Laurin-, Tridecan-, Myristin-, Pentadecan-, Palmitin-, Heptadecan-, Stearin-, Nonadecan-, Arachin- und Behensäure. Ungesättigte Vertreter sind beispielsweise Lauroelein-, Myristolein-, Palmitolein-, Petroselaidin-, Öl-, Elaidin-, Ricinol-, Linol-, Linolaidin-, Linolen- Gadolein-, Arachidon und Erucasäure. Auch Mischungen der Methylester dieser Säuren sind geeignet. Besonders bevorzugt ist die Verwendung solcher PIT-Emulsionen, die Methylester aus der Gruppe Methyloleat, Methylpamitat, Methylstearat und/oder Methylpelargonat enthalten. Es können aber auch Methylester auf Basis natürlicher Fettsäuremischungen eingesetzt werden, wie sie beispielsweise aus Lein-, Kokos-, Palm-, Palmkem-, Oliven- , Ricinus-, Rüb-, Soja- oder Sonnenblumenölen (bei Raps- und Sonnenblumenöl jeweils neue und alte Züchtungen) erhalten werden.

Geeignete Verbindungen der Gruppe b) sind native Öle pflanzlichen Ursprungs. Es handelt sich dabei im Wesentlichen um Triglyceridmischungen, wobei das Glycerin mit längerkettigen Fettsäuren jeweils vollständig verestert ist. Besonders geeignete pflanzliche Öle sind ausgewählt aus der Gruppe Erdnuß-, Kokos- und/oder Sonnenblumenöl.

Erdnußöl enthält durchschnittlich (bezogen auf Fettsäure) 54 Gew.-% Ölsäure, 24 Gew.-Linolsäure, 1 Gew.-% Linolensäure, 1 Gew.-% Arachinsäure, 10 Gew.-% Palmitinsäure, sowie 4 Gew.-% Stearinsäure. Der Schmelzpunkt beträgt 2 bis 3 °C.

Leinöl enthält typischerweise 5 Gew.-% Palmitin-, 4 Gew.-% Stearin-, 22 Gew.-% Öl-, 17 Gew.-% Linol- und 52 Gew.-% Linolensäure. Die Iodzahl liegt im Bereich von 155 bis 205, Die Verseifungszahl ist 188 bis 196 und der Schmelzpunkt liegt bei etwa - 20 °C. Kokosöl enthält an Fettsäuren etwa 0,2 bis 1 Gew.-% Hexan-, 5 bis 8 Gew.-% Octan-, 6 bis 9 Gew.-% Decan-, 45 bis 51 Gew.-% Laurin-, 16 bis 19 Gew.-% Myristin-, 9 bis 11 Gew.-% Palmitin-, 2 bis 3 Gew.-% Stearin-, weniger als 0,5 Gew.-% Behen-, 8 bis 10 Gew.-% Öl- und bis 1 Gew.-% Linolsäure. Die Iodzahl liegt im Bereich von 7,5 bis 9,5 , die Verseifungszahl liegt bei 0,88 bis 0,90. Der Schmelzpunkt liegt bei 20 bis 23 °C. Olivenöl enthält überwiegend Ölsäure (vergl. Lebensmittelchem. Gerichtl. Chem., 39, 112 bis 114, 1985). Palmöl enthält als Fettsäurekomponenten etwa 2 Gew.-% Myristin-, 42 Gew.-% Palmitin-, 5 Gew.-% Stearin-, 41 Gew.-% Öl-, 10 Gew.-% Linolsäure. Palmkernöl ist typischerweise in Bezug auf das Fettsäurespektrum wie folgt zusammengesetzt: 9 Gew.-% Capron/Capryl/Caprin-, 50 Gew.-% Laurin-, 15 Gew.-% Myristin-, 7 Gew.-% Palmitin-, 2 Gew.-% Stearin-, 15 Gew.-% Öl- und 1 Gew.-% Linolsäure.

Rapsöl enthält als Fettsäurekomponenten typischerweise etwa 48 Gew.-% Erucasäure, 15 Gew.-% Ölsäure, 14 Gew.-% Linolsäure, 8 Gew.-% Linolensäure, 5 Gew.-% Icosensäure, 3 Gew.-% Palmitinsäure, 2 Gew.-% Hexadecensäure und 1 Gew.-% Docosadiensäure. Rapsöl aus neuer Züchtung ist bezüglich der ungesättigten Anteile angereichert. Typische Fettsäureanteile sind hier Erucasäure 0,5 Gew.-%, Ölsäure 63 Gew.-%, Linolsäure 20 Gew.-%, Linolensäure 9 Gew.-%, Icosensäure 1 Gew.-%, Palmitinsäure 4 Gew.-%, Hexadecensäure 2 Gew.-% und Docosadiensäure 1 Gew.-%.

Ricinusöl besteht zu 80 bis 85 Gew.-% aus dem Glycerid der Ricinolsäure, daneben sind zu etwa 7 Gew.-% Glyceride der Öl-, zu 3 Gew.-% Glyceride der Linol- und zu etwa 2 Gew.-% die Glyceride der Palmitin- und der Stearinsäure enthalten.

Sojaöl enthält zu 55 bis 65 Gew.-% der Gesamtfettsäuren mehrfach ungesättigte Säuren, insbesondere Linol- und

Linolensäure. Ähnlich ist die Situation beim Sonnenblumenöl , dessen typisches Fettsäurespektrum, bezogen auf Gesamtfettsäure wie folgt aussieht: ca. 1 Gew.-% Myristin-, 3 bis 10 Gew.-% Palmitin-, 14 bis 65 Gew.-% Öl- und 20 bis 75 Gew.-% Linolsäure.

Alle obigen Angaben über den Fettsäureanteile in den Triglyceriden sind bekanntermaßen abhängig von der Qualität der Rohstoffe und können daher zahlenmäßig schwanken. Besonders bevorzugt sind solche PIT-Emulsionen, die Nährstoffe der Gruppe b), ausgewählt aus der Gruppe Kokosöl. Sonnenblumenöl und/oder Rapsöl enthalten.

Wichtige Bestandteile der erfindungsgemäß verwendeten PIT-Emulsionen sind die eingesetzten Emulgatoren bzw. Emulgatorensysteme. Vorzugsweise werden als Emulgatoren nichtionische Emulgatoren, insbesondere ethoxylierte Fettalkohole und Fettsäuren eingesetzt. Zur Ausbildung von PIT-Emulsionen ist es vorteilhaft ein zweikomponentiges Emulgatorsystem, enthalten einen hydrophilen Emulgator (A) und einen hydrophoben Coemulgator (B) einzusetzen. Als hydrophiler nichtionische Emulgatoren (A) eignen sich Stoffe, die einen HLB-Wert von etwa 8 bis 18 aufweisen. Unter dem HLB-Wert (Hydrophil-Lipophil-Balance) soll ein Wert verstanden werden, der gemäß

$$HLB = (100-L) / 5$$

errechnet werden kann, wobei L der Gewichtsanteil der lipophilen Gruppen, d.h. der Fettalkyl- oder Fettacylgruppen in Prozent in den Ethylenoxidanlagerungsprodukten ist.

Fettalkoholethoxylate im Sinne der erfindungsgemäßen Lehre folgen der allgemeinen Formel (II)

$$R^3\text{-O-}(CH_2CH_2O)_n\text{-H} \qquad \text{(II)}$$

wobei $R^3$ für einen linearen oder verzweigten, gesättigten oder ungesättigten Alkylrest mit 6 bis 24 Kohlenstoffatomen steht und n eine Zahl von 1 bis 50 bedeutet. Besonders bevorzugt sind solche Verbindungen der Formel (II), in der n für eine Zahl von 1 bis 35 und insbesondere von 1 bis 15 steht. Besonders bevorzugt sind weiterhin solche Verbindungen der Formel (II), in der $R^3$ für einen Alkylrest mit 16 bis 22 Kohlenstoffatomen steht.

Die Verbindungen der Formel (II) werden in an sich bekannter Weise durch Umsetzung von Fettalkoholen mit Ethylenoxid unter Druck, ggf. in Gegenwart saurer oder basischer Katalysatoren erhalten. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Fettsäureethoxylate, die ebenfalls als Emulgatorkomponente (A) in Betracht kommen, folgen vorzugsweise der Formel (III),

$$R^4CO_2(CH_2CH_2O)_mH \qquad \text{(III)}$$

in der $R^4$ für einen linearen oder verzweigten Alkylrest mit 12 bis 22 Kohlenstoffatomen und m für Zahlen von 5 bis 50 und vorzugsweise 15 bis 35 steht. Typische Beispiele sind Anlagerungsprodukte von 20 bis 30 Mol Ethylenoxid an Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelen'schen Oxosynthese anfallen. Vorzugsweise werden Anlagerungsprodukte von 20 bis 30 Mol Ethylenoxid an Fettsäuren mit 16 bis 18 Kohlenstoffatomen eingesetzt.

Partialglyceride, die als Emulgatorkomponente (B) in Betracht kommen, folgen vorzugsweise der Formel (IV),

$$\begin{array}{l} CH_2O(CH_2CH_2O)_x\text{-}COR^5 \\ | \\ CH\text{-}O(CH_2CH_2O)_yH \\ | \\ CH_2O(CH_2CH_2O)_z\text{-}H \end{array} \qquad (IV)$$

in der CO $R^5$ für einen linearen oder verzweigten Acylrest mit 12 bis 22 Kohlenstoffatomen und x, y und z in Summe für 0 oder für Zahlen von 1 bis 50, vorzugsweise 15 bis 35 steht. Typische Beispiele für im Sinne der Erfindung geeignete Partialglyceride sind Laurinsäuremonoglycerid, Kokosfettsäuremonoglycerid, Palmitinsäuremonoglycerid, Stearinsäuremonoglycerid, Isostearinsäuremonoglycerid, Ölsäuremonoglycerid und Talgfettsäuremonoglycerid sowie deren Addukte mit 5 bis 50 und vorzugsweise 20 bis 30 Mol Ethylenoxid. Vorzugsweise werden Monoglyceride bzw. technische Mono/Diglyceridgemische mit überwiegendem Monoglyceridanteil der Formel (IV) eingesetzt, in der CO $R^5$ für einen linearen Acylrest mit 16 bis 18 Kohlenstoffatomen steht.

Üblicherweise werden Emulgatormischungen eingesetzt, die die Komponenten (A) und (B) im Gewichtsverhältnis 10 : 90 bis 90 : 10, vorzugsweise 25 : 75 bis 75 : 25 und insbesondere 40 : 60 bis 60 : 40 enthalten.

Als weitere geeignete Emulgatoren kommen beispielsweise nichtionogene Tenside aus einer der folgenden Gruppen in Frage:

(I) Anlagerungsprodukte von 2 bis 30 Mol Ethylenoxid und/ oder 0 bis 5 Mol Propylenoxid an lineare Fettalkohole mit 8 bis 22 C-Atomen;
(II) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(III) Alkylmono- und -oligoglycoside mit 8 bis 22 Kohlenstoffatomen im Alkylrest und deren ethoxylierte Analoga;
(IV) Anlagerungsprodukte von 15 bis 60 Mol Ethylenoxid an Ricinusöl und/oder gehärtetes Ricinusöl;
(V) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(VI) Anlagerungsprodukte von 2 bis 15 Mol Ethylenoxid an Ricinusöl und/oder gehärtetes Ricinusöl;
(VII) Partialester auf Basis linearer, verzweigter, ungesättigter bzw. gesättigter $C_{12/22}$-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(VIII) Wollwachsalkohole;
(IX) Polyalkylenglycole.

Die Anlagerungsprodukte von Ethylenoxid und/oder von Propylenoxid an Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

Zur Auswahl geeigneter Emulgatorsysteme kann es zweckmäßig sein, rechnerisch die Ermittlung der PIT des jeweiligen Systems durchzuführen. Insbesondere gilt das aber auch für potentielle Optimierungen in der Auswahl der Emulgatoren beziehungsweise Emulgatorsysteme und ihrer Anpassung an die durch sonstige Überlegungen zum technischen Handeln vorgegebene Auswahl und Abmischung von wäßriger Phase einerseits und Typ der Ölphase andererseits. Entsprechendes Fachwissen ist aus an sich ganz anderen Bereichen, insbesondere aus dem Bereich der Kosmetika-Herstellung, entwickelt worden. Verwiesen wird insbesondere auf die Veröffentlichung TH.Förster, W. von Rybinski, H.Tesmann und A.Wadle "*Calculation of optimum emulsifier mixtures for phase inversion emulsification*", in International Journal of Cosmetic Science 16, 84-92 (1994). Dargestellt ist hier im einzelnen wie auf rechnerischem Weg für vorgegebene 3-Komponenten-Systeme aus einer Ölphase, einer Wasserphase und einem Emulgator auf der Basis des für die Ölphase charakteristischen EACN-Wertes (equivalent alkane carbon number) der Temperaturbereich der Phaseninversion (PIT) über die CAPICO-Methode (calculation of phase inversion in concentrates) errechnet werden kann. Diese Veröffentlichung Förster et al. bezieht insbesondere wiederum wesentliche Literatur für den hier angeschnittenen Themenkomplex ein, die im Zusammenhang mit der Offenbarung dieser Veröffentlichung Förster et al. zu sehen ist. Im einzelnen wird dann anhand zahlreicher Beispiele dargestellt, wie mittels der CAPICO-Methode im Rahmen des EACN-Konzepts die Auswahl und Optimierung der Emulgatoren/Emulgatorsysteme zur optimalen Einstellung vorgegebener Werte für den Temperaturbereich der Phaseninversion zugänglich wird.

Die erfindungsgemäß verwendeten PIT-Emulsionen enthalten vorzugsweise von 20 bis 90 Gew.-% Wasser,

insbesondere von 30 bis 80 Gew.-% und ganz besonders bevorzugt von 30 bis 60 Gew.-%. Der Rest auf 100 Gew.-% entfällt auf die Ölphase sowie Emulgatoren und ggf. weitere Hilfs- und Zusatzstoffe. Die Ölphase selbst ist vorzugsweise in Mengen von 10 bis 80 Gew.-%, insbesondere von 40 bis 70 Gew.-% enthalten. Dabei enthält die Ölphase vorzugsweise ausschließlich die Komponenten a) oder b) bzw. Mischungen dieser Komponenten. Besonders bevorzugt ist die Verwendung solcher Emulsionen, die Öl- und Wasserphase im Gewichtsverhältnis von 1 : 1 enthalten. Die Emulgatoren, bzw. Emulgatorensysteme sind vorzugsweise in Mengen von 1 bis 25 Gew.-%, insbesondere in Mengen von 5 bis 20 und besonders bevorzugt in Mengen von 5 bis 15 Gew.-% enthalten. Die erfindungsgemäß verwendeten Emulsionen weisen vorzugsweise Phaseninversionstemperaturen im Bereich von 20 bis 95 °C und insbesondere von 40 bis 95 °C auf.

Die beschriebenen PIT-Emulsionen können erfindungsgemäß in Fermentationsprozessen aller Art eingesetzt werden. Dabei können alle dem Fachmann bekannten Verfahrensausgestaltungen, z.B. Batch- oder Fed-Batch sowie kontinuierliche Fermentation verwendet werden. Auch sind alle dem Fachmann bekannten Fermentersysteme einsetzbar. Zu den Einzelheiten siehe *Crueger*, Seiten 50 bis 70. Die Verwendung der Mikroemulsionen ist auch nicht auf bestimmte Mikroorganismen begrenzt, vielmehr lassen sich die Emulsionen zur Herstellung oder Umwandlung aller dem Fachmann durch Fermentation bekannten Verbindungen einsetzten. Neben den klassischen Fermentationsverfahren, die überwiegend zu Synthese von Antibiotika eingesetzt werden, (vergl. *Crueger,* Seiten 197 bis 242) eignen sich die beschriebenen Emulsionen aber auch zum Einsatz bei mikrobiellen Transformationen ("Bioconversion"), z.B. der Transformation von Steroiden und Sterinen, von Antibiotika und Pestiziden oder der Herstellung von Vitaminen (vergl. *Crueger*, Seiten 254 bis 273). Bevorzugt ist aber die Verwendung in Fermentationsprozessen zur Herstellung von Antibiotika, beispielsweise Chephalosporinen, Tylosin oder Erythromycin.

In der Regel werden die Emulsionen der wäßrigen Fermentationsbrühe, welche die Mikroorganismen sowie die Stickstoffquelle und Spurenelemente und ggf. weitere Hilfsstoffe, insbesondere Entschäumer, enthält, in geeigneter Weise zudosiert. Als Stickstoffquellen kommen beispielsweise in Betracht: Pepton, Hefe- oder Malzextrakt, Maisquellwasser, Harnstoff oder Lecithine. Die Spurenelemente können in Form anorganischer Salze anwesend sein, beispielsweise Natrium- oder Kaliumnitrat, Ammoniumnitrat, Ammoniumsulfat, Eisensulfat etc.. Es kann auch vorteilhaft sein, den PIT-Emulsionen selbst weitere Zusatzstoffe, wie Entschäumer oder Stickstoffquellen zuzusetzen.

**Beispiele**

Es wurden verschiedene Emulsionen durch Mischen der Ausgangsstoffe und Erwärmen der Mischung über die PIT-Temperatur sowie nachfolgender Abkühlung auf Raumtemperatur (20 °C) hergestellt (Verfahren nach DE 38 19193 A1). Die PIT-Temperatur wurde durch Leitfähigkeitsmessung ermittelt. Die Tröpfchengröße wurde mit einem Coulter N4 Plus Submicron Particle Sizer gemessen. Der Meßwinkel betrug 90 °. Die Ergebnisse sind in Tabellen 1a und 1b aufgeführt. Diese Emulsionen eignen sich beispielsweise als alleinige Nährstoffquelle für Fermentationsprozessen und können direkt der wäßrigen Fermentationsbrühe zugesetzt werden.

Tabelle 1a

| | Gew.- % | Gew.- % | Gew.- % | Gew.- % | Gew.- % | Gew.- % | Gew.- % | Gew.- % |
|---|---|---|---|---|---|---|---|---|
| Rapsöl | 34 | 45 | 45 | 45 | 45 | 40 | 40 | 45 |
| Wasser | 55 | 37 | 37 | 37 | 37 | 44 | 44 | 37 |
| Ricinusöl, ethoxyliert mit 40 Mol EO pro Mol Ricinusöl | 5 | 3 | | | | | | |
| Behenylakohol + 10 EO | 2,3 | | 3 | 5 | 4 | 3 | 3 | 3 |
| Glycerinoleat | 3,7 | | | 2 | 1 | | 1 | |
| Hydriertes Ricinusöl, ethoxyliert mit 7 Mol EO pro Mol Ricinusöl | | 15 | 15 | 11 | 13 | 13 | 12 | 13 |
| *PIT* | *84°C* | *83°C* | *68°C* | *61°C* | *65°C* | *72°C* | *57°C* | *75°C* |
| *Tröpfchengröße* | *318 nm* | *293nm* | *230 nm* | *187nm* | *195nm* | *229nm* | *175nm* | *269 nm* |

Tabelle 1b

| | Gew.-% | Gew.-% | Gew.-% | Gew.- % | Gew.- % |
|---|---|---|---|---|---|
| Methyloleat | 45 | 45 | | | |
| Sonnenblumenöl | | | | 45 | 40 |
| Kokosöl | | | 34 | | |
| Wasser | 45 | 45 | 55 | 37 | 44 |
| C16/18 Fettalkohol + 12 EO | 5 | 7 | | | |
| Ricinusöl, ethoxyliert mit 40 Mol EO pro Mol Ricinusöl | | | 4 | | |
| Behenylalkohol + 10 EO | | | 2,8 | 3 | 3 |
| Glycerinmonostearat | | 3 | | | |
| Glycerinoleat | | | 4,2 | | |
| Hydriertes Ricinusöl, ethoxyliert mit 7 Mol EO pro Mol Ricinusöl | | | | 15 | 12 |
| C16-Fettalkohol + 6 EO | 5 | | | | |
| *PIT* | *73°C* | *74°C* | *61°C* | *68°C* | *57°C* |
| *Tröpfchengröße* | *201 nm* | *216 nm* | *156 nm* | *219 nm* | *180 nm* |

**Patentansprüche**

**1.** Verwendung von Öl-in-Wasser-Emulsionen, enthaltend mindesten Wasser, Emulgatoren sowie ein Ölphase, die eine oder mehrere Verbindungen, ausgewählt aus den Gruppen

a) der Fettsäurealkylester und/oder
b) der Triglyceride pflanzlichen Ursprungs

enthält, **dadurch gekennzeichnet, dass** die Emulsion nach dem Phaseninversionstemperatur-Verfahren hergestellt wird und eine Tröpfchengröße von 50 bis 400 nm aufweist, in Fermentationsverfahren.

**2.** Verwendung nach Anspruch 1, **dadurch gekennzeichnet, dass** die Ölphase als Komponente a) Fettsäuremethylester enthält.

**3.** Verwendung nach Anspruch 1 oder 2, **dadurch gekennzeichnet, dass** Emulsionen verwendet werden, die eine mittlere Tröpfchengröße im Bereich von 100 bis 300 nm, vorzugsweise 180 bis 300 nm und insbesondere von 160 bis 250 nm aufweisen.

**4.** Verwendung nach den Ansprüchen 1 bis 3, **dadurch gekennzeichnet, dass** Emulsionen verwendet werden, die Wasser in Mengen von 20 bis 90 Gew.-%, vorzugsweise von 30 bis 80 Gew.-% und insbesondere von 30 bis 60 Gew.-% enthalten.

**5.** Verwendung nach den Ansprüchen 1 bis 4, **dadurch gekennzeichnet, dass** Emulsionen verwendet werden, die Ölphase in Mengen von 10 bis 80 Gew.-%, vorzugsweise 40 bis 70 Gew.-% enthalten.

**6.** Verwendung nach den Ansprüchen 1 bis 5, **dadurch gekennzeichnet, dass** Emulsionen verwendet werden, die Fettsäuremethylester der Formel (I) enthalten,

$$R^1\text{-COO-}R^2 \quad (I)$$

wobei $R^1$ für eine Alkylrest mit 6 bis 22 C-Atomen steht und $R^2$ einen Methylrest bedeutet.

7. Verwendung nach den Ansprüchen 1 bis 6, **dadurch gekennzeichnet, dass** Emulsionen verwendet werden, die in der Ölphase Methyloleat, Methylpalmitat, Methylstearat und/oder Methylpelargonat enthalten.

8. Verwendung nach den Ansprüchen 1 bis 7, **dadurch gekennzeichnet, dass** Emulsionen verwendet werden, die in der Ölphase Kokos-, Sonnenblumen- und/oder Rapsöl enthalten.

9. Verwendung nach den Ansprüchen 1 bis 8, **dadurch gekennzeichnet, dass** Emulsionen verwendet werden, die ein Emulgatorsystem, enthaltend hydrophile Emulgatoren mit Hydrophil-Lipophil-Balance Werten von 8 bis 18 in Kombination mit hydrophoben Coemulgatoren enthalten.

10. Verwendung nach Anspruch 9, **dadurch gekennzeichnet, dass** Emulsionen verwendet werden, deren Emulgatorsysteme Mengenverhältnisse zwischen hydrophilen Emulgatoren und Coemulgatoren von 10 : 90 bis 90 : 10 aufweisen.

11. Verwendung nach den Ansprüchen 1 bis 10, **dadurch gekennzeichnet, dass** Emulsionen verwendet werden, die Emulgatoren in Mengen von 1 bis 25 Gew.-%, vorzugsweise in Mengen von 5 bis 20 Gew.-% und insbesondere in Mengen von 5 bis 15 Gew.-% enthalten.

## Claims

1. The use of o/w emulsions containing at least water, emulsifiers and an oil phase containing one or more compounds selected from the groups of

   a) fatty acid alkyl esters and/or
   b) triglycerides of vegetable origin I

   **characterized in that** the emulsion is produced by the phase inversion temperature method and has a droplet size of 50 to 400 nm,
   in fermentation processes.

2. The use claimed in claim 1, **characterized in that** the oil phase contains fatty acid methyl esters as component a).

3. The use claimed in claim 1 or 2, **characterized in that** emulsions with a mean droplet size of 100 to 300 nm, preferably 180 to 300 nm and more particularly 160 to 250 nm are used.

4. The use claimed in claims 1 to 3, **characterized in that** emulsions containing water in quantities of 20 to 90% by weight, preferably 30 to 80% by weight and more particularly 30 to 60% by weight are used.

5. The use claimed in claims 1 to 4, **characterized in that** emulsions containing the oil phase in quantities of 10 to 80% by weight and preferably 40 to 70% by weight are used.

6. The use claimed in claims 1 to 5, **characterized in that** emulsions containing fatty acid methyl esters of formula (I):

$$R^1\text{-COO-}R^2 \quad (I)$$

   in which $R^1$ is a $C_{6-22}$ alkyl group and $R^2$ is a methyl group,
   are used.

7. The use claimed in claims 1 to 6, **characterized in that** emulsions containing methyl oleate, methyl palmitate, methyl stearate and/or methyl pelargonate in the oil phase are used.

8. The use claimed in claims 1 to 7, **characterized in that** emulsions containing coconut oil, sunflower oil and/or rapeseed oil in the oil phase are used.

9. The use claimed in claims 1 to 8, **characterized in that** emulsions containing an emulsifier system containing

hydrophilic emulsifiers with hydrophilic/lipophilic balance values of 8 to 18 in combination with hydrophobic co-emulsifiers are used

10. The use claimed in claim 9, **characterized in that** emulsions of which the emulsifier systems have quantity ratios between hydrophilic emulsifiers and co-emulsifiers of 10:90 to 90:10 are used.

11. The use claimed in claims 1 to 10, **characterized in that** emulsions containing emulsifiers in quantities of 1 to 25% by weight, preferably in quantities of 5 to 20% by weight and more particularly in quantities of 5 to 15% by weight are used.

## Revendications

1. Utilisation d'émulsions huile dans eau, contenant au moins de l'eau, des émulsifiants ainsi qu'une phase huileuse qui contient un ou plusieurs composés, choisis dans les groupes

   a) des esters alkyliques d'acides gras et/ou
   b) des triglycérides d'origine végétale

   **caractérisée en ce que**
   l'émulsion est produite selon la méthode de la température d'inversion de phase et présente des gouttelettes mesurant 50 à 400 nm, lors d'un procédé de fermentation.

2. Utilisation selon la revendication 1,
   **caractérisée en ce que**
   la phase huileuse contient des esters méthyliques d'acides gras en tant que composant a).

3. Utilisation selon la revendication 1 ou 2,
   **caractérisée en ce qu'**
   on utilise des émulsions dont les gouttelettes présentent une taille moyenne de l'ordre de 100 à 300 nm, de préférence de 180 à 300 nm et en particulier de 160 à 250 nm.

4. Utilisation selon les revendications 1 à 3,
   **caractérisée en ce qu'**
   on utilise des émulsions qui contiennent de l'eau en quantité de 20 à 90 % en poids, de préférence de 30 à 80 % en poids et, en particulier, de 30 à 60 % en poids.

5. Utilisation selon les revendications 1 à 4,
   **caractérisée en ce qu'**
   on utilise des émulsions qui contiennent la phase huileuse en quantité de 10 à 80 % en poids, de préférence de 40 à 70 % en poids.

6. Utilisation selon les revendications 1 à 5,
   **caractérisée en ce qu'**
   on utilise des émulsions qui contiennent des esters méthyliques d'acides gras de formule (I)

$$R^1\text{-COO-}R^2 \qquad (I)$$

   dans laquelle $R^1$ représente un radical alkyle comportant 6 à 22 atomes de C, et $R^2$ un radical méthyle.

7. Utilisation selon les revendications 1 à 6,
   **caractérisée en ce qu'**
   on utilise des émulsions qui contiennent, dans la phase huileuse, de l'oléate de méthyle, du palmitate de méthyle, du stéarate de méthyle et/ou du pélargonate de méthyle.

8. Utilisation selon les revendications 1 à 7,

**caractérisée en ce qu'**
on utilise des émulsions qui contiennent, dans la phase huileuse, de l'huile de coco, de tournesol et/ou de colza.

9. Utilisation selon les revendications 1 à 8,
**caractérisée en ce qu'**
on utilise des émulsions qui renferment un système émulsifiant contenant des émulsifiants hydrophiles ayant une valeur de HLB (hydrophillipophil-balance) de 8 à 18 en combinaison avec des co-émulsifiants hydrophobes.

10. Utilisation selon la revendication 9,
**caractérisée en ce qu'**
on utilise des émulsions dont les systèmes émulsifiants ont des rapports quantitatifs entre les émulsifiants hydrophiles et les co-émulsifiants de 10/90 à 90/10.

11. Utilisation selon les revendications 1 à 10,
**caractérisée en ce qu'**
on utilise des émulsions qui contiennent des émulsifiants en quantité de 1 à 25 % en poids, de préférence de 5 à 20 % en poids et, en particulier, de 5 à 15 % en poids.